(19) Europäisches Patentamt
European Patent Office
Office européen des brevets

(11) EP 0 728 734 B1

(12) EUROPÄISCHE PATENTSCHRIFT

(45) Veröffentlichungstag und Bekanntmachung des Hinweises auf die Patenterteilung:
**09.09.1998 Patentblatt 1998/37**

(51) Int. Cl.[6]: **C07C 209/36**, C07C 221/00, C07C 213/02, C07C 225/20, C07C 253/30, C07C 255/58

(21) Anmeldenummer: **96102416.3**

(22) Anmeldetag: **17.02.1996**

(54) **Verfahren zur Herstellung aromatischer Amine**

Process for the preparation of aromatic amines

Procédé pour la préparation d'amines aromatiques

(84) Benannte Vertragsstaaten:
**CH DE FR GB IT LI**

(30) Priorität: **23.02.1995 DE 19506278**

(43) Veröffentlichungstag der Anmeldung:
**28.08.1996 Patentblatt 1996/35**

(73) Patentinhaber:
**HOECHST AKTIENGESELLSCHAFT**
**65929 Frankfurt am Main (DE)**

(72) Erfinder:
- **Beller, Matthias, Dr**
  **D-65510 Idstein (DE)**
- **Tafesh, Ahmed, Dr.**
  **D-65779 Kelkheim (DE)**
- **Kohlpaintner, Christian, Dr.**
  **TX 78413522, Kenith Circle (US)**
- **Naumann, Christoph, Dr.**
  **D-65527 Niedernhausen (DE)**

(56) Entgegenhaltungen:
**EP-A- 0 362 037** **EP-A- 0 369 864**
**WO-A-92/06067**

- **JOURNAL OF NATURAL GAS CHEMISTRY, Bd. 4, Nr. 4, April 1995 Seiten 393-399, XP 000195871 XIA CHUN-GU 'Ruthenium-TPPTS catalyzed reduction of nitrobenzene to aniline '**
- **JOURNAL OF THE CHEMICAL SOCIETY, CHEMICAL COMMUNICATIONS, 1994 LETCHWORTH GB, Seiten 863-864, XP 000195897 NAGAVELLI P. 'Palladium Complex-Potassium Carbonate-catalysed Reductive Carbonylation of Mono- and Di-nitroaromatic Compounds'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.3/3.4

## Beschreibung

Die vorliegende Erfindung betrifft ein gegenüber dem Stand der Technik verbessertes Verfahren zur Herstellung aromatischer Amine. Ein häufig beschrittener Weg zur Herstellung aromatischer Amine besteht darin, eine aromatische Nitroverbindung unter Verwendung von Trägerkatalysatoren, die beispielsweise Nickel oder Palladium enthalten, zu hydrieren. Hierbei gelangt der Trägerkatalysator entweder als Festbett angeordnet oder in Form einer Aufschlämmung (Suspension) zur Anwendung. Ein Nachteil bei der Verwendung von als Festbett angeordneten Trägerkatalysatoren ist, daß man bei Durchführung dieses Verfahrens an spezielle Reaktorbehälter, beispielsweise Rohrreaktoren mit einem innen angeordneten Kühlsystem, gebunden ist. Setzt man den Trägerkatalysator als Aufschlämmung ein, so ergeben sich während der Reaktion Probleme mit der kontrollierten Abführung von Wärme und zudem muß nach der Beendigung der Umsetzung der Trägerkatalysator, beispielsweise durch eine aufwendige Filtration, abgetrennt werden.

Bei Durchführung dieser beiden Verfahren muß stets darauf geachtet werden, daß Katalysatorreste - hervorgerufen durch Abrieb des als Festbett angeordneten Katalysators oder durch unzureichende Filtration der Katalysatoraufschlämmung - nicht in das Reaktionsprodukt gelangen. Es ist nämlich bekannt, daß selbst sehr geringe Mengen der Trägerkatalysatoren zu empfindlichen Störungen bei der Weiterverarbeitung der Reaktionsprodukte führen können. Darüber hinaus lassen sowohl Palladium- als auch Nickel-Trägerkatalysatoren hinsichtlich der Selektivität der Hydrierung aromatischer Verbindungen Wünsche offen. Sie führen nämlich bei einer Reihe von empfindlichen Substituenten zu unerwünschten Reaktionen, indem sie diese Substituenten entweder abspalten oder sie hydrieren.

In der WO-A-9206067 ist ein Verfahren zur Herstellung aromatischer Amine mit Hilfe eines wasserunlöslichen Palladium-Katalysators beschrieben.

Die EP-A-0 369 864 beschreibt ein Verfahren zur Herstellung von aromatischen Aminen aus Nitroverbindungen mit Hilfe eines Rhodium und Ruthenium Katalysators. Die beschriebenen Phosphin-Liganden sind nicht wasserlöslich.

Aus der EP-A-0 362 037 sind lösliche Phosphine bekannt, die zur Trennung von Rhodium-Katalysatoren verwendet werden können. Die Katalysatoren werden nicht zur Reduktion von Nitroverbindungen eingesetzt.

In J. Chem. Soc., Chem. Commun., 1994 Seiten 863 bis 864 ist ein Verfahren zur reduktiven Carbonylierung aromatischer Mono- und Dinitroverbindungen beschrieben. Die aromatischen Mono- und Dinitroverbindungen lassen sich in einem Benzol-Methanol-Gemisch mit Kohlenmonoxid in Anwesenheit katalytischer Mengen von 1,3-Bis(diphenylphosphino)propanpalladiumdichlorid und Kaliumcarbonat zu Urethanen umsetzen. Die Umsetzung liefert die gewünschten Urethane (Carbamate) als Hauptprodukt. Daneben bilden sich auch Amine und gegebenenfalls Azo- und Azoxyverbindungen. Im Hinblick auf eine Herstellung von Aminen erscheint das Verfahren wenig geeignet, da die maximale Aminausbeute bei Verwendung von p-Nitroanisol als Ausgangsprodukt lediglich bei 40 % liegt. In allen anderen Fällen liegt die Aminausbeute, wie aus Table 1 hervorgeht, noch niedriger.

Es besteht daher ein Bedarf, ein Verfahren zur Herstellung aromatischer Amine bereitzustellen, das die vorstehend genannten Nachteile vermeidet und sich auf einfache Weise durchführen läßt. Darüber hinaus soll das Verfahren nicht nur übliche aromatische Amine sondern auch solche aromatischen Amine, die empfindliche Substituenten aufweisen, zugänglich machen.

Diese Aufgabe wird überraschenderweise durch das erfindungsgemäße Verfahren zur Herstellung aromatischer Amine gelöst. Es ist dadurch gekennzeichnet, daß man eine aromatische Nitroverbindung in einem aus Wasser und einem wasserunlöslichen organischen Lösungsmittel bestehenden Lösungsmittelgemisch mittels eines Palladium und ein wasserlösliches Phosphin enthaltenden Katalysators mit Kohlenmonoxid unter einem Druck von 5 bis 300 bar und bei einer Temperatur von 50 bis 200°C umsetzt und die wäßrige Phase und die organische Phase trennt.

Das erfindungsgemäße Verfahren besitzt mehrere Vorteile. Es ist zum einen nicht an die Verwendung spezieller Reaktorbehälter (Rohrreaktoren mit innen angeordneter Kühlung) gebunden und vermeidet zum anderen eine zeitraubende und aufwendige Abtrennung des Katalysators nach Beendigung der Reaktion. Es sei an dieser Stelle darauf hingewiesen, daß der durch Filtration abgetrennte Trägerkatalysator in hochaktiver Form vorliegt und deshalb mit besonderer Sorgfalt zu handhaben ist. Diese Schwierigkeiten treten bei Durchführung des erfindungsgemäßen Verfahrens nicht auf, da der aus Palladium und einem wasserlöslichen Phosphin gebildete Katalysator in Wasser gelöst vorliegt und durch eine einfache Abtrennung der wäßrigen Phase aus dem Reaktionsgemisch entfernt werden kann. Die wäßrige, den Katalysator enthaltende Phase kann anschließend wieder in die Reaktion eingesetzt werden, ohne daß besondere Schutzmaßnahmen bezüglich der Handhabung der wäßrigen, den aktiven Katalysator enthaltenden Lösung erforderlich sind.

Ferner bereitet das Abführen der bei der erfindungsgemäßen Umsetzung freiwerdenden Reaktionswärme keine Probleme, da die Umsetzung in Anwesenheit eines eine besonders hohe Wärmekapazität aufweisenden Wärmepuffers, nämlich Wasser, abläuft und zudem überschüssige Wärme durch Verdampfung von Wasser wirkungsvoll abgeleitet werden kann. Das Problem der Temperaturführung vereinfacht sich auch deshalb, weil die bei der Reduktion der aromatischen Nitroverbindung mittels CO und unter Bildung von $CO_2$ freigesetzte Wärmemenge geringer ist als die bei einer katalytischen Hydrierung einer aromatischen Nitroverbindung unter Bildung von Wasser entstehende Wärmemenge.

Als aromatische Nitroverbindung wird eine Verbindung der allgemeinen Formel $R^1R^2R^3ArNO_2$, worin $R^1$, $R^2$, $R^3$ gleich oder verschieden sind und für Wasserstoff, einen Alkyl-oder Alkoxyrest mit 1 bis 6 Kohlenstoffatomen, einen halogenierten Alkylrest mit 1 bis 4 Kohlenstoffatomen, ein Halogen, -OH, -CHO, -CO-$R^4$, -$CO_2R^4$, -CONH$R^4$, -CON($R^4$)$_2$, -S$R^4$, wobei $R^4$ ein Rest mit 1 bis 6 Kohlenstoffatomen ist, -CN oder -CH = CH-$R^5$, wobei $R^5$ Wasserstoff, ein Alkylrest mit 1 - 4 Kohlenstoffatomen oder ein Phenylrest ist, stehen und Ar einen aromatischen Rest bedeutet, als aromatische Nitroverbindung eingesetzt.

In einer Reihe von Fällen setzt man eine Verbindung $R^1R^2R^3ArNO_2$, worin $R^1$, $R^2$ und $R^3$ für Wasserstoff, einen Alkyl- oder Alkoxyrest mit 1 bis 4 Kohlenstoffatomen, einen halogenierten Alkylrest mit 1 bis 2 Kohlenstoffatomen, ein Halogen, -OH, -CO-$R^4$, -CN oder -CH = CH-$R^5$ stehen, als aromatische Nitroverbindung einsetzt.

Halogen, -OH, -CO-$R^4$, -CN und -CH = CH-$R^5$ stellen problematische Substituenten dar, die entweder im Verlauf einer katalytischen Hydrierung abgespalten oder reduziert werden, beziehungsweise im Falle der OH-Gruppe desaktivierend wirken. Beabsichtigt man aromatische Amine, die einen oder mehreren dieser Substituenten aufweisen sollen, herzustellen, so setzt man eine Verbindung $R^1R^2R^3ArNO_2$, worin wenigstens einer der Reste $R^1$, $R^2$ und $R^3$ für ein Halogen, -OH, -CO-$R^4$, -CN oder -CH = CH-$R^5$ steht, oder einer oder zwei der Reste $R^1$, $R^2$ und $R^3$ für ein Halogen, -OH, -CO-$R^4$, -CN oder -CH = CH-$R^5$ steht und insbesondere einer oder zwei der Reste $R^1$, $R^2$ und $R^3$ für -Cl, -Br, - OH, -CO-$R^4$, wobei $R^4$ ein Alkylrest mit 1 bis 4 Kohlenstoffatomen oder ein Phenylrest ist, -CN oder -CH = CH-$R^5$, wobei $R^5$ Wasserstoff, ein Alkylrest mit 1 bis 4 Kohlenstoffatomen oder ein Phenylrest ist, steht, als aromatische Nitroverbindung ein.

Man setzt eine Verbindung $R^1R^2R^3ArNO_2$, worin Ar für einen aromatischen Rest mit 6 bis 20 Kohlenstoffatomen, insbesondere für einen Phenyl-, Naphthyl-, Phenanthryl-, Biphenyl- oder Binaphthylrest, bevorzugt für einen Phenyl-, Naphthyl- oder Biphenylrest, besonders bevorzugt für einen Phenylrest steht, als aromatische Nitroverbindung ein.

Die Umsetzung findet in einem 2-phasigen System, das einerseits aus Wasser und andererseits aus einem wasserunlöslichen organischen Lösungsmittel besteht, statt. Man setzt einen aromatischen Kohlenwasserstoff, einen aliphatischen Kohlenwasserstoff, einen chlorierten aromatischen oder aliphatischen Kohlenwasserstoff oder ein Gemisch derselben, beispielsweise Toluol, ortho-Xylol, meta-Xylol, para-Xylol, Gemische isomerer Xylole, Ethylbenzol, Mesitylen, Chlortoluol, Chlorbenzol, Dichlorbenzol oder Gemische der vorstehend genannten Lösungsmittel als wasserunlösliches organisches Lösungsmittel ein.

Der für die Durchführung der Reaktion benötigte Katalysator enthält Palladium und ein wasserlösliches Phosphin. Üblicherweise verwendet man Palladium in Form eines wasserlöslichen Palladiumsalzes, insbesondere Palladiumchlorid, Palladiumnitrat, Palladiumacetat oder Palladiumsulfat.

Bei Ausübung des Verfahrens ist man an kein bestimmtes Verhältnis von Palladium zu Phosphin gebunden. Üblicherweise kann man Palladium und das wasserlösliche Phosphin im Molverhältnis 1:0,2 bis 1:100, insbesondere 1:1 bis 1:50, bevorzugt 1:2 bis 1:20 einsetzen.

Verwendet man ein wasserlösliches Diphosphin, so genügt es in der Regel, Palladium und das Diphosphin im Molverhältnis 1:1 bis 1:10, insbesondere 1:2 bis 1:6 einzusetzen.

Verwendet man ein wasserlösliches Monophosphin, so genügt es in den meisten Fällen, Palladium und das Monophosphin im Molverhältnis 1:2 bis 1:20, insbesondere 1:4 bis 1:12 einzusetzen.

Gut geeignet als wasserlösliches Phosphin ist ein -$SO_3M$ Gruppen enthaltendes Phosphin und/oder Diphosphin, worin M für Wasserstoff, Ammonium, ein einwertiges Metall oder das Äquivalent eines mehrwertigen Metalls, insbesondere für Ammonium oder ein Alkalimetall, bevorzugt Natrium oder Kalium, besonders bevorzugt Natrium steht. Es lassen sich auch Gemische verschiedener Phosphine oder Gemische von Phosphinen unterschiedlichen Sulfonierungsgrades mit gutem Erfolg verwenden.

In einer Reihe von Fällen hat es sich bewährt, ein 1 bis 6, insbesondere 1 bis 3 - $SO_3M$ Gruppen enthaltendes Triarylphosphin, worin der Arylrest gleich oder verschieden ist und für Phenyl oder Naphthyl steht, oder ein Gemisch derartiger Triarylphosphine, insbesondere ein vergleichsweise leicht zugängliches 1 bis 3-$SO_3M$ Gruppen enthaltendes Triphenylphosphin, einzusetzen.

Gute Ergebnisse lassen sich erzielen, indem man als wasserlösliches Phosphin ein Phosphin der allgemeinen Formel (A)

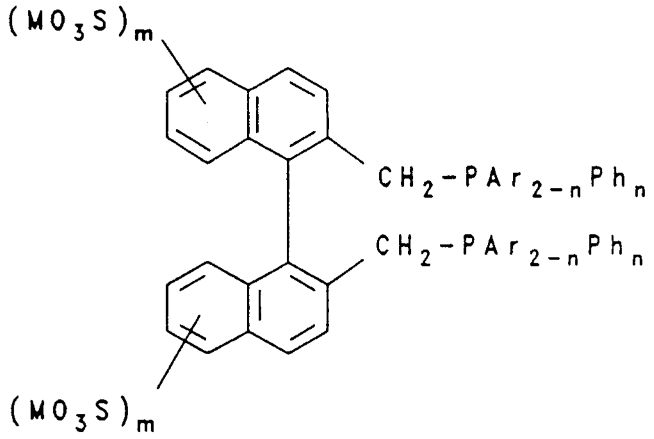

(A)

worin Ar für m-$C_6H_4SO_3M$, M für Wasserstoff, für Ammonium, ein einwertiges Metall oder das Äquivalent eines mehrwertigen Metalls und Ph für einen Phenylrest steht, m gleich oder verschieden ist und den Wert 1 oder 2 hat und n gleich oder verschieden ist und 0, 1 oder 2 bedeutet, oder ein Gemisch derartiger Phosphine einsetzt.

Das erfindungsgemäße Verfahren läßt sich mit besonders gutem Erfolg ausüben, indem man als wasserlösliches Phosphin ein Phosphin der allgemeinen Formel (B)

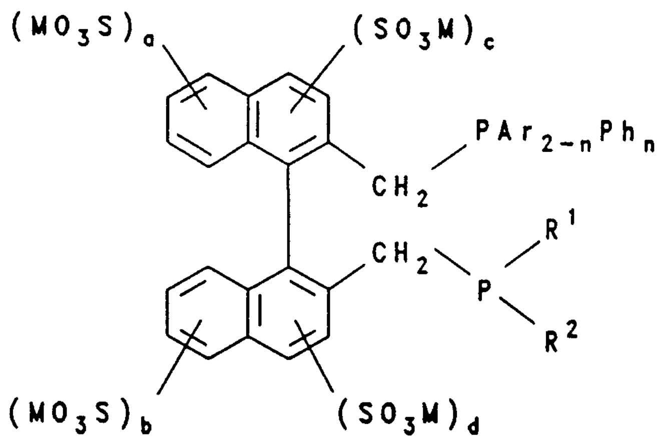

(B)

worin M für Wasserstoff, Ammonium, ein einwertiges Metall oder das Äquivalent eines mehrwertigen Metalls steht, a, b, c und d jeweils 0 oder 1 ist, mit der Maßgabe, daß a + b + c + d einer ganzen Zahl von 1 bis 4 entspricht, Ar für einen m-($MSO_3$)-$C_6H_4$-Rest steht und M die vorstehende Bedeutung hat, n für 0, 1 oder 2, Ph für einen Phenylrest steht, $R^1$ und $R^2$ gleich oder verschieden sind und für einen Alkyrest mit 1 bis 10 Kohlenstoffatomen oder einen cycloaliphatischen Rest mit 5 bis 10 Kohlenstoffatomen im Ring stehen, oder ein Gemisch derartiger Phosphine einsetzt.

Wegen ihrer guten Eignung sind Phosphine der Formel (B), worin $R^1$ und $R^2$ jeweils für einen Cyclohexylrest stehen, gesondert zu nennen. Es lassen sich auch Gemische dieser Phosphine verwenden.

An dieser Stelle sei erwähnt, daß es sich bei den Phosphinen der Formel (B) um neue Phosphine handelt. Diese Phosphine und ihre Herstellung sind Gegenstand einer am selben Tag wie die vorliegende Patentanmeldung eingereichten deutschen Patentanmeldung (Aktenzeichen 195 06 279.5).

Das Verfahren läßt sich auch bei einem Druck < 5 bar und einem Druck > 300 bar durchführen. Drücke unterhalb 5 bar führen zu einer deutlichen Erhöhung der Reaktionszeit, Drücke oberhalb 300 bar erfordern üblicherweise entsprechend ausgelegte Druckreaktoren.

In den meisten Fällen hat sich ein Druck von 10 bis 200, insbesondere 50 bis 150 bar als ausreichend erwiesen.

Man kann das Verfahren auch bei Temperaturen < 50°C ausüben, muß hierbei allerdings deutlich verlängerte Reaktionszeiten in Kauf nehmen. Temperaturen > 200°C führen zu einer erheblichen Beschleunigung der Umsetzung, wobei auch die Bildung von Nebenprodukten begünstigt werden kann. Üblicherweise führt man das Verfahren bei 70

bis 160, insbesondere 80 bis 150°C durch. Diese Temperaturbereiche haben sich für ein Vielzahl von Fällen als ausreichend erwiesen.

Nach Beendigung der Umsetzung wird das Reaktionsgemisch abgekühlt und entspannt. Anschließend wird die organische Phase, die das Wertprodukt enthält, und die wäßrige Phase, die den Katalysator enthält, voneinander getrennt. Die leichte Abtrennbarkeit des Katalysators stellt einen Vorteil des Verfahrens dar, da eine aufwendige Abtrennung und Rückgewinnung des Katalysators entfällt. Die abgetrennte wäßrige Phase kann, gegebenenfalls nach Zusatz von Palladium und/oder wasserlöslichem Phosphin, wieder in die Reaktion eingesetzt werden.

Die vorliegende Erfindung betrifft ferner neue zur Durchführung des Verfahrens geeignete Katalysatoren, die Palladium und das Phosphin der Formel (B) im Molverhältnis 1:1 bis 1:10, insbesondere 1:2 bis 1:6 enthalten. Die Katalysatoren enthalten Palladium und insbesondere das Phosphin der Formel (B), worin $R^1$ und $R^2$ jeweils für einen Cyclohexylrest stehen, im Molverhältnis 1:1 bis 1:10, insbesondere 1:2 bis 1:6.

Zur Durchführung des Verfahrens kann man die einzelnen Komponenten des Katalysators nämlich das Palladium, beispielsweise in Form eines wasserlöslichen Salzes, und das wasserlösliche Phosphin in die Reaktion einsetzen.

Falls gewünscht, kann man den Katalysator durch Zusammenführen eines wasserlöslichen Pd-Salzes und des wasserlöslichen Phosphins in wäßriger Lösung vorab herstellen und als wäßrige Lösung oder in isolierter Form in das Verfahren einsetzen.

Die nachfolgenden Beispiele beschreiben die Erfindung näher, ohne sie darauf zu beschränken.

## Experimenteller Teil

### Beispiel 1a

Herstellung von 4-Chlor-3-aminoacetophenon

Eine entgaste Lösung von 40 mmol 4-Chlor-3-nitroacetophenon (Einsatzstoff) und 60 ml Xylol werden in einen Autoklaven (Volumen: 200 ml) eingefüllt. Als Phosphin werden 5 mmol TPPTS (in Form von 9,2 g einer wäßrigen Lösung, die 0,546 mol TPPTS /kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.

Anschließend wird CO bis auf einen Druck von 120 bar aufgepreßt und das Gemisch unter Rühren auf 100°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt, gegebenenfalls durch Kristallisation oder Chromatographie, gereinigt wird.

### Beispiel 1b

Herstellung von 4-Chlor-3-aminoacetophenon

Eine entgaste Lösung von 40 mmol 4-Chlor-3-nitroacetophenon (Einsatzstoff) und 60 ml Xylol werden in einen Autoklaven (Volumen: 200 ml) eingefüllt. Als Phosphin werden 2,5 mmol BINAS (in Form von 14,4 g einer wäßrigen Lösung, die 0,173 mol BINAS/kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.

Anschließend wird CO bis auf einen Druck von 120 bar aufgepreßt und das Gemisch unter Rühren auf 90°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt gegebenenfalls durch Kristallisation oder Chromatographie gereinigt wird.

Beispiel 2a

Herstellung von 2-Chlor-5-trifluormethylanilin

Eine entgaste Lösung von 40 mmol 2-Chlor-5-trifluormethylnitrobenzol (Einsatzstoff) und 40 ml Xylol werden in einen Autoklaven (Volumen: 200 ml) eingefüllt. Als Phosphin werden 5,0 mmol TPPTS (in Form von 9,2 g einer wäßrigen Lösung, die 0,546 mol TPPTS /kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.

Anschließend wird CO bis auf einen Druck von 120 bar aufgepreßt und das Gemisch unter Rühren auf 100°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt gegebenenfalls durch Kristallisation oder Chromatographie gereinigt wird.

Beispiel 2b

Herstellung von 2-Chlor-5-trifluormethylanilin

Eine entgaste Lösung von 40 mmol 2-Chlor-5-trifluormethylnitrobenzol (Einsatzstoff) und 40 ml Xylol werden in einen Autoklaven (Volumen: 200 ml) eingefüllt. Als Phosphin werden 3,0 mmol BINAS (in Form von 17,3 g einer wäßrigen Lösung, die 0,173 mol BINAS /kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Anschließend wird, wie in Beispiel 2a angegeben, gearbeitet.

Beispiel 3a

Herstellung von 4-Chlor-3-aminobenzonitril

Eine entgaste Lösung von 40 mmol 4-Chlor-3-nitrobenzonitril (Einsatzstoff) und 40 ml Xylol werden in einen Autoklaven (Volumen: 200 ml) eingefüllt. Als Phosphin werden 5,0 mmol TPPTS (in Form von 9,2 g einer wäßrigen Lösung, die 0,546 mol TPPTS/kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.

Anschließend wird CO bis auf einen Druck von 120 bar aufgepreßt und das Gemisch unter Rühren auf 100°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt gegebenenfalls durch Kristallisation oder Chromatographie gereinigt wird.

Beispiel 3b

Herstellung von 4-Chlor-3-aminobenzonitril

Eine entgaste Lösung von 40 mmol 4-Chlor-3-nitrobenzonitril (Einsatzstoff) und 40 ml Xylol werden in einen Autoklaven (Volumen: 200 ml) eingefüllt. Als Phosphin werden 2,5 mmol BINAS (in Form von 14,4 g einer wäßrigen Lösung, die 0,173 mol BINAS/kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Anschließend wird, wie in Beispiel 3a angegeben, gearbeitet.

Beispiel 4a

Herstellung von 4-Chloranilin

Eine entgaste Lösung von 40 mmol 4-Chlornitrobenzol (Einsatzstoff) und 40 ml Xylol werden in einen Autoklaven (Volumen: 200 ml) eingefüllt. Als Phosphin werden 5,0 mmol TPPTS (in Form von 9,2 g einer wäßrigen Lösung, die 0,546 mol TPPTS/kg Lösung enthält) sowie 2,4 g (60 mmol) NaOH, 23,8 ml $H_2O$ und 1,0 mmol $PdCl_2$ zugesetzt. Der pH-Wert beträgt 10,5 bis 11,0.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.
Anschließend wird CO bis auf einen Druck von 120 bar aufgepreßt und das Gemisch unter Rühren auf 100°C erwärmt. Die Reaktionsezit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt gegebenenfalls durch Kristallisation oder Chromatographie gereinigt wird.

Beispiel 4b

Herstellung von 4-Chloranilin

Eine entgaste Lösung von 35 mmol 4-Chlornitrobenzol (Einsatzstoff) und 40 ml Xylol werden in einem Autoklaven (Volumen: 200 ml) eingefüllt. Als Phosphin werden 2,57 mmol BINAS (in Form von 14,85 g einer wäßrigen Lösung, die 0,173 mol BINAS/kg Lösung) enthält sowie 2,4 g (60 mmol) NaOH, 22,0 ml $H_2O$ und 0,85 mmol $PdCl_2$ zugesetzt. Der pH-Wert beträgt 10,5 bis 11,0.

Anschließend wird, wie in Beispiel 4a angegeben, gearbeitet.

Beispiel 5a

Herstellung von 5-Chlor-2-hydroxyanilin

Eine entgaste Lösung von 10 mmol 5-Chlor-2-hydroxynitrobenzol (Einsatzstoff) und 40 ml Xylol werden in einem Autoklaven (Volumen: 200 ml) eingefüllt. Als Phosphin werden 0,25 mmol TPPTS (in Form von 4,6 g einer wäßrigen Lösung, die 0,546 mol TPPTS/kg Lösung enthält) sowie 0,6 g (15 mmol) NaOH, 41 ml $H_2O$ und 0,25 mmol $PdCl_2$ zugesetzt. Der pH-Wert beträgt 10 bis 10,5.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.
Anschließend wird CO bis auf einen Druck von 120 bar aufgepreßt und das Gemisch unter Rühren auf 100°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt, gegebenenfalls durch Kristallisation oder Chromatographie, gereinigt wird.

Beispiel 5b

Herstellung von 5-Chlor-2-hydroxyanilin

Eine entgaste Lösung von 10 mmol 5-Chlor-2-hydroxynitrobenzol (Einsatzstoff) und 40 ml Xylol werden in einen Autoklaven (Volumen: 200 ml) eingefüllt. Als Phosphin werden 0,125 mmol BINAS (in Form von 0,72 g einer wäßrigen Lösung, die 0,173 mol BINAS/kg Lösung enthält) sowie 0,6 g (15 mmol) NaOH, 41 ml $H_2O$ und 0,25 mmol $PdCl_2$ zugesetzt. Der pH-Wert beträgt 10 bis 11.

Anschließend wird, wie in Beispiel 5a angegeben, gearbeitet.

Beispiel 5c

Herstellung von 5-Chlor-2-hydroxyanilin

Eine entgaste Lösung von 10 mmol 5-Chlor-2-hydroxynitrobenzol (Einsatzstoff) und 40 ml Xylol werden in einen Autoklaven (Volumen: 200 ml) eingefüllt. Als Phosphin werden 0,125 mmol Ligand A (in Form von 1,25 g einer wäßrigen Lösung, die 0,1 mol Ligand A/kg Lösung enthält) sowie 0,6 g (15 mmol) NaOH, 41 ml $H_2O$ und 0,25 mmol $PdCl_2$ zugesetzt. Der pH-Wert beträgt 10,5 bis 11,0.

Anschließend wird, wie in Beispiel 5a angegeben, gearbeitet.

Der als Phosphin verwendete Ligand A führt im Vergleich zu den Beispielen 5a und 5b zu einer signifikanten Erhöhung der Ausbeute. Vergleiche auch die nachfolgende Tabelle.

Beispiel 6

Herstellung von 3-Aminostyrol

Eine entgaste Lösung von 40 mmol 3-Nitrostyrol (Einsatzstoff) und 40 ml Xylol werden in einen Autoklaven (Volumen: 200 ml) eingefüllt. Als Phosphin werden 2,0 mmol BINAS (in Form von 11,56 g einer wäßrigen Lösung, die 0,173 mol BINAS/kg Lösung enthält) sowie 1,92 g (48 mmol) NaOH, 23 ml $H_2O$ und 0,8 mmol $PdCl_2$ zugesetzt. Der pH-Wert beträgt 10,0 bis 10,5.

Der Autoklav wird geschlossen, evakuiert und mit Stickstoff befüllt. Das Evakuieren und Befüllen mit Stickstoff wird zweimal wiederholt, dann wird CO eingefüllt, evakuiert und CO erneut eingefüllt. Das Evakuieren und Einfüllen von CO wird zweimal wiederholt.

Anschließend wird CO bis auf einen Druck von 120 bar aufgepreßt und das Gemisch unter Rühren auf 100°C erwärmt. Die Reaktionszeit beträgt 20 Stunden. Anschließend wird auf Raumtemperatur abgekühlt, der Autoklav entleert und die organische Phase von der Wasserphase getrennt. Die organische Phase wird zur Abtrennung von Spuren von Palladium filtriert und unter Vakuum eingeengt, wobei das gewünschte Produkt gegebenenfalls durch Kristallisation oder Chromatographie gereinigt wird.

Nachstehend finden sich die Erklärungen zu verwendeten Abkürzungen TPPTS, BINAS und Ligand A.

TPPTS steht für ein Gemisch von Natriumsalzen sulfonierter Triphenylphosphine, enthaltend Natriumsalze monosulfonierter, disulfonierter und trisulfonierter Triphenylphosphine. Die Herstellung derartiger Salze ist in DE 26 27 354 beschrieben.

BINAS steht für Natriumsalze sulfonierter 2,2'-Bis(diphenylphosphinomethyl)-1,1'-binapthaline der allgemeinen Formel (A)

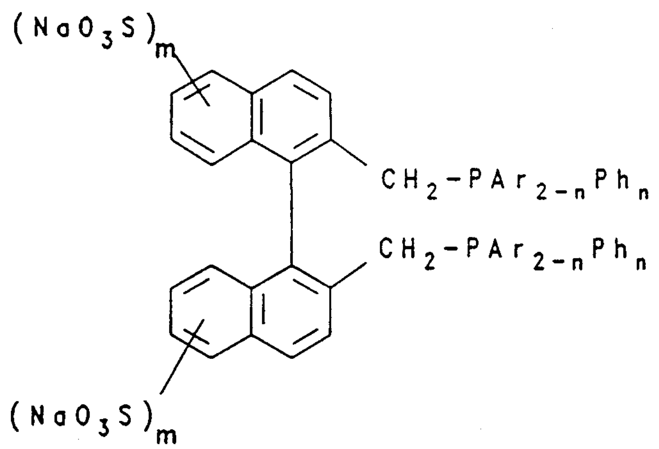

(A)

in der Ar für m-$C_6H_4$ $SO_3Na$ und Ph für den Phenylrest steht, m gleich oder verschieden ist und den Wert 1 oder 2 hat und n gleich oder verschieden ist und 0, 1 oder 2 bedeutet.

Die Herstellung dieser Salze bzw. Salzgemische ist in der EP 0 571 819 beschrieben.

Ligand A steht für ein Phosphin der Formel (B)

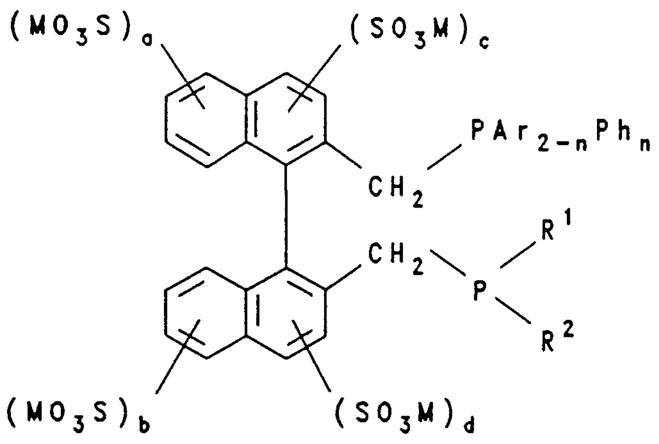

(B)

worin M für Na steht, a, b, c und d jeweils 0 oder 1 ist, mit der Maßgabe, daß a + b + c + d einer ganzen Zahl von 1 bis 4 entspricht, Ar für einen m-($NaSO_3$)-$C_6H_4$-Rest steht, n für 0, 1 oder 2, Ph für einen Phenylrest steht und $R^1$ und $R^2$ jeweils für einen Cyclohexylrest stehen.

Die Herstellung dieser Verbindung bzw. eines Gemisches dieser Verbindungen ist in der am selben Tag wie die vorliegende Patentanmeldung eingereichten deutschen Patentanmeldung (Aktenzeichen 19506279.5) beschrieben.

Die Ergebnisse der vorstehend beschriebenen Beispiele sind in der nachfolgenden Tabelle zusammengestellt. Die in den Beispielen beschriebenen Umsetzungen basieren im wesentlichen auf ersten Versuchsbefunden. Bei entsprechender Optimierung der Reaktionsdurchführung dürften sich daher die Ergebnisse, insbesondere die Ausbeuten, noch verbessern lassen.

Tabelle

| Beispiel | Einsatzstoff | Phosphin | Endprodukt | Ausbeute |
|---|---|---|---|---|
| 1a<br>1b | 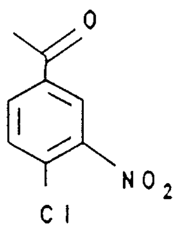 | TPPTS<br>BINAS | 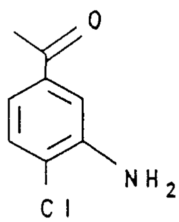 | 65 %<br>70 % |
| 2a<br>2b | 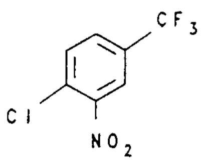 | TPPTS<br>BINAS | 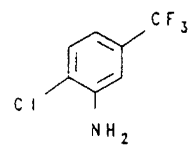 | 45 %<br>85 % |
| 3a<br>3b | 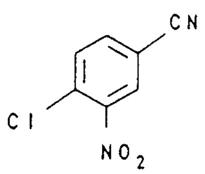 | TPPTS<br>BINAS | 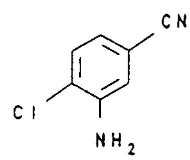 | 60 %<br>60 % |
| 4a<br>4b | 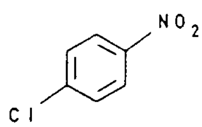 | TPPTS<br>BINAS | 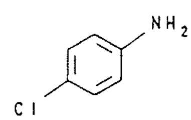 | 40 %<br>50 % |
| 5a<br>5b<br>5c | 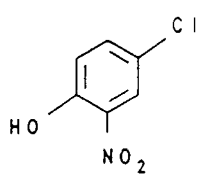 | TPPTS<br>BINAS<br>Ligand A | 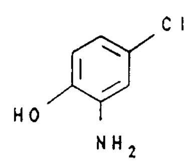 | 5 %<br>10 %<br>50 % |

| Beispiel | Einsatzstoff | Phosphin | Endprodukt | Ausbeute |
|---|---|---|---|---|
| 6 | 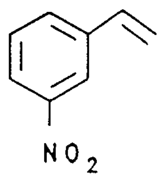 | BINAS | 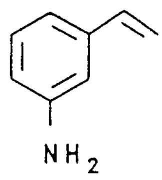 | 50 % |

**Patentansprüche**

1. Verfahren zur Herstellung aromatischer Amine, dadurch gekennzeichnet, daß man eine aromatische Nitroverbindung der allgemeinen Formel $R^1R^2R^3ArNO_2$, worin $R^1$, $R^2$ und $R^3$ gleich oder verschieden sind und Wasserstoff, einen Alkyl- oder Alkoxyrest mit 1 bis 6 Kohlenstoffatomen, einen halogenierten Alkylrest mit 1 bis 4 Kohlenstoffatomen, ein Halogen, -OH, -CHO, -CO-$R^4$, -$CO_2R^4$, -CONH$R^4$, -CON($R^4$)$_2$, -S$R^4$, wobei $R^4$ ein Rest mit 1 bis 6 Kohlenstoffatomen ist, -CN oder -CH = CH-$R^5$, wobei $R^5$ Wasserstoff, ein Alkylrest mit 1 - 4 Kohlenstoffatomen oder ein Phenylrest ist, stehen und Ar einen aromatischen Rest bedeutet, in einem aus Wasser und einem wasserunlöslichem organischen Lösungsmittel bestehenden Lösungsmittelgemisch mittels eines Palladium und ein wasserlösliches Phosphin enthaltenden Katalysators mit Kohlenmonoxid unter einem Druck von 5 bis 300 bar und bei einer Temperatur von 50 bis 200°C umsetzt, und die wäßrige Phase und die organische Phase trennt.

2. Verfahren nach Anspruch 1, dadurch gekennzeichnet, daß man eine Verbindung $R^1R^2R^3ArNO_2$, worin $R^1$, $R^2$ und $R^3$ für Wasserstoff, einen Alkyl- oder Alkoxyrest mit 1 bis 4 Kohlenstoffatomen, einen halogenierten Alkylrest mit 1 bis 2 Kohlenstoffatomen, ein Halogen, - OH, -CO-$R^4$, -CN oder -CH = CH-$R^5$ stehen, als aromatische Nitroverbindung einsetzt.

3. Verfahren nach Anspruch 1 oder 2, dadurch gekennzeichnet, daß man eine Verbindung $R^1R^2R^3ArNO_2$, worin wenigstens einer der Reste $R^1$, $R^2$ und $R^3$ für ein Halogen, -OH, -CO-$R^4$, -CN oder -CH = CH-$R^5$ steht, als aromatische Nitroverbindung einsetzt.

4. Verfahren nach einem oder mehreren der Ansprüche 1 bis 3, dadurch gekennzeichnet, daß man eine Verbindung $R^1R^2R^3ArNO_2$, worin einer oder zwei der Reste $R^1$, $R^2$ und $R^3$ für ein Halogen, -OH, -CO-$R^4$, -CN oder -CH = CH-$R^5$ steht, als aromatische Nitroverbindung einsetzt.

5. Verfahren nach einem oder mehreren der Ansprüche 1 bis 4, dadurch gekennzeichnet, daß man eine Verbindung $R^1R^2R^3ArNO_2$, worin einer oder zwei der Reste $R^1$, $R^2$ und $R^3$ für -Cl, -Br, -OH, -CO-$R^4$, wobei $R^4$ ein Alkylrest mit 1 bis 4 Kohlenstoffatomen oder ein Phenylrest ist, -CN oder -CH = CH-$R^5$, wobei $R^5$ Wasserstoff, ein Alkylrest mit 1 bis 4 Kohlenstoffatomen oder ein Phenylrest ist, als aromatische Nitroverbindung einsetzt.

6. Verfahren nach einem oder mehreren der Ansprüche 1 bis 5, dadurch gekennzeichnet, daß man eine Verbindung $R^1R^2R^3ArNO_2$, worin Ar für einen aromatischen Rest mit 6 bis 20 Kohlenstoffatomen steht, als aromatische Nitroverbindung einsetzt.

7. Verfahren nach einem oder mehreren der Ansprüche 1 bis 6, dadurch gekennzeichnet, daß man eine Verbindung $R^1R^2R^3ArNO_2$, worin Ar für einen Phenyl-, Naphthyl-, Phenanthryl-, Biphenyl- oder Binaphthylrest steht, als aromatische Nitroverbindung einsetzt.

8. Verfahren nach einem oder mehreren der Ansprüche 1 bis 7, dadurch gekennzeichnet, daß man eine Verbindung $R^1R^2R^3ArNO_2$, worin Ar für einen Phenyl-, Naphthyl- oder Biphenylrest steht, als aromatische Nitroverbindung einsetzt.

9. Verfahren nach einem oder mehreren der Ansprüche 1 bis 8, dadurch gekennzeichnet, daß man eine Verbindung $R^1R^2R^3ArNO_2$, worin Ar für einen Phenylrest steht, als aromatische Nitroverbindung einsetzt.

10. Verfahren nach einem oder mehreren der Ansprüche 1 bis 9, dadurch gekennzeichnet, daß man einen aromatischen Kohlenwasserstoff, einen aliphatischen Kohlenwasserstoff, einen chlorierten aromatischen oder aliphatischen Kohlenwasserstoff oder ein Gemisch derselben als wasserunlösliches organisches Lösungsmittel einsetzt.

11. Verfahren nach einem oder mehreren der Ansprüche 1 bis 10, dadurch gekennzeichnet, daß man Palladium in Form eines wasserlöslichen Palladiumsalzes, insbesondere als $PdCl_2$, $Pd(NO_3)_2$, $Pd(acetat)_2$ oder $PdSO_4$ einsetzt.

12. Verfahren nach einem oder mehreren der Ansprüche 1 bis 11, dadurch gekennzeichnet, daß man ein $-SO_3M$ Gruppen enthaltendes Phosphin, worin M für Wasserstoff, Ammonium, ein einwertiges Metall oder das Äquivalent eines mehrwertigen Metalls steht, als wasserlösliches Phosphin einsetzt.

13. Verfahren nach einem oder mehreren der Ansprüche 1 bis 12, dadurch gekennzeichnet, daß man ein $-SO_3M$ Gruppen enthaltendes Phosphin, worin M für Ammonium oder ein Alkalimetall, insbesondere Natrium oder Kalium, bevorzugt Natrium steht, einsetzt.

14. Verfahren nach einem oder mehreren der Ansprüche 1 bis 13, dadurch gekennzeichnet, daß man ein 1 bis 6, insbesondere 1 bis 3 $-SO_3M$ Gruppen enthaltendes Triarylphosphin, worin der Arylrest gleich oder verschieden ist und für Phenyl oder Naphthyl steht, oder ein Gemisch derartiger Triarylphosphine einsetzt.

15. Verfahren nach einem oder mehrere der Ansprüche 1 bis 14, dadurch gekennzeichnet, daß man ein 1 bis 3 $-SO_3M$ Gruppen enthaltendes Triphenylphosphin einsetzt.

16. Verfahren nach einem oder mehreren der Ansprüche 1 bis 13, dadurch gekennzeichnet, daß man ein Phosphin der allgemeinen Formel (A)

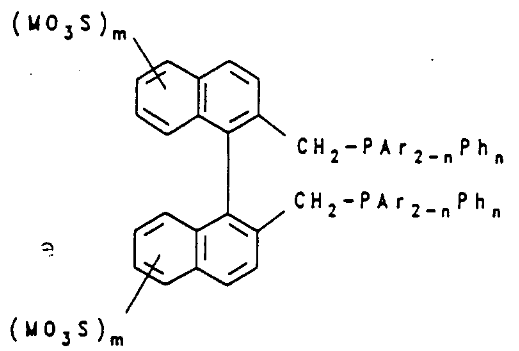

(A)

worin Ar für $m-C_6H_4SO_3M$, M für Wasserstoff, für Ammonium, ein einwertiges Metall oder das Äquivalent eines mehrwertigen Metalls und Ph für einen Phenylrest steht, m gleich oder verschieden ist und den Wert 1 oder 2 hat und n gleich oder verschieden ist und 0, 1 oder 2 bedeutet, oder ein Gemisch derartiger Phosphine einsetzt.

17. Verfahren nach einem oder mehrere der Ansprüche 1 bis 13, dadurch gekennzeichnet, daß man ein Phosphin der allgemeinen Formel (B)

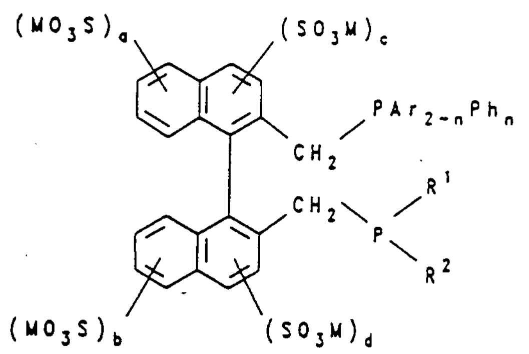

(B)

worin M für Wasserstoff, Ammonium, ein einwertiges Metall oder das Äquivalent eines mehrwertigen Metalls steht, a, b, c und d jeweils 0 oder 1 ist, mit der Maßgabe, daß a + b + c + d einer ganzen Zahl von 1 bis 4 entspricht, Ar für einen m-$(MSO_3)$-$C_6H_4$-Rest steht und M die vorstehende Bedeutung hat, n für 0, 1 oder 2, Ph für einen Phenylrest steht, $R^1$ und $R^2$ gleich oder verschieden sind und für einen Alkylrest mit 1 bis 10 Kohlenstoffatomen oder einen cycloaliphatischen Rest mit 5 bis 10 Kohlenstoffatomen im Ring stehen, oder ein Gemisch derartiger Phosphine einsetzt.

18. Verfahren nach einem oder mehreren der Ansprüche 1 bis 13 und 17, dadurch gekennzeichnet, daß man ein Phosphin der Formel (B), worin $R^1$ und $R^2$ jeweils für einen Cyclohexylrest stehen, einsetzt.

19. Katalysator zur Durchführung des Verfahrens nach einem oder mehreren der Ansprüche 1 bis 13 und 17 bis 18, dadurch gekennzeichnet, daß der Katalysator Pd und das Phosphin der Formel (B) im Molverhältnis 1:1 bis 1:10, insbesondere 1:2 bis 1:6 enthält.

20. Katalysator zur Durchführung des Verfahrens nach einem oder mehreren der Ansprüche 1 bis 13 und 17 bis 18, dadurch gekennzeichnet, daß der Katalysator Pd und das Phosphin der Formel (B), worin $R^1$ und $R^2$ jeweils für einen Cyclohexylrest stehen, im Molverhältnis 1:1 bis 1:10, insbesondere 1:2 bis 1:6 enthält.

## Claims

1. A process for preparing aromatic amines, which comprises reacting an aromatic nitro compound of the formula $R^1R^2R^3ArNO_2$, where $R^1$, $R^2$ and $R^3$ are identical or different and are each hydrogen, an alkyl or alkoxy radical having from 1 to 6 carbon atoms, a halogenated alkyl radical having from 1 to 4 carbon atoms, a halogen, -OH, -CHO, -CO-$R^4$, -$CO_2R^4$, -CONH$R^4$, -CON$(R^4)_2$, -S$R^4$, where $R^4$ is a radical having from 1 to 6 carbon atoms, -CN or -CH=CH-$R^5$, where $R^5$ is hydrogen, an alkyl radical having 1-4 carbon atoms or a phenyl radical, and Ar is an aromatic radical, with carbon monoxide in a solvent mixture comprising water and a water-insoluble organic solvent by means of a catalyst comprising palladium and a water-soluble phosphine under a pressure of from 5 to 300 bar and at a temperature of from 50 to 200°C, and separating the aqueous phase and the organic phase.

2. The process as claimed in claim 1, wherein the aromatic nitro compound used is a compound $R^1R^2R^3ArNO_2$, where $R^1$, $R^2$ and $R^3$ are hydrogen, an alkyl or alkoxy radical having from 1 to 4 carbon atoms, a halogenated alkyl radical having 1 or 2 carbon atoms, a halogen, -OH, -CO-$R^4$, -CN or -CH=CH-$R^5$.

3. The process as claimed in claim 1 or 2, wherein the aromatic nitro compound used is a compound $R^1R^2R^3ArNO_2$, where at least one of the radicals $R^1$, $R^2$ and $R^3$ is a halogen, -OH, -CO-$R^4$, -CN or -CH=CH-$R^5$.

4. The process as claimed in one or more of claims 1 to 3, wherein the aromatic nitro compound used is a compound $R^1R^2R^3ArNO_2$, where one or two of the radicals $R^1$, $R^2$ and $R^3$ are a halogen, -OH, -CO-$R^4$, -CN or -CH=CH-$R^5$.

5. The process as claimed in one or more of claims 1 to 4, wherein the aromatic nitro compound used is a compound $R^1R^2R^3ArNO_2$, where one or two of the radicals $R^1$, $R^2$ and $R^3$ are -Cl, -Br, -OH, -CO-$R^4$, where $R^4$ is an alkyl radical having from 1 to 4 carbon atoms or is a phenyl radical, -CN or -CH=CH-$R^5$, where $R^5$ is hydrogen, an alkyl radical having from 1 to 4 carbon atoms or a phenyl radical.

6. The process as claimed in one or more of claims 1 to 5, wherein the aromatic nitro compound used is a compound $R^1R^2R^3ArNO_2$, where Ar is an aromatic radical having from 6 to 20 carbon atoms.

7. The process as claimed in one or more of claims 1 to 6, wherein the aromatic nitro compound used is a compound $R^1R^2R^3ArNO_2$, where Ar is a phenyl, naphthyl, phenanthryl, biphenyl or binaphthyl radical.

8. The process as claimed in one or more of claims 1 to 7, wherein the aromatic nitro compound used is a compound $R^1R^2R^3ArNO_2$, where Ar is a phenyl, naphthyl or biphenyl radical.

9. The process as claimed in one or more of claims 1 to 8, wherein the aromatic nitro compound used is a compound $R^1R^2R^3ArNO_2$, where Ar is a phenyl radical.

10. The process as claimed in one or more of claims 1 to 9, wherein the water-insoluble organic solvent used is an aromatic hydrocarbon, an aliphatic hydrocarbon, a chlorinated aromatic or aliphatic hydrocarbon or a mixture thereof.

11. The process as claimed in one or more of claims 1 to 10, wherein palladium is used in the form of a water-soluble palladium salt, in particular as $PdCl_2$, $Pd(NO_3)_2$, $Pd(acetate)_2$ or $PdSO_4$.

12. The process as claimed in one or more of claims 1 to 11, wherein the water-soluble phosphine used is a phosphine containing $-SO_3M$ groups, where M is hydrogen, ammonium, a monovalent metal or the equivalent of a polyvalent metal.

13. The process as claimed in one or more of claims 1 to 12, wherein a phosphine containing $-SO_3M$ groups, where M is ammonium or an alkali metal, in particular sodium or potassium, preferably sodium, is used.

14. The process as claimed in one or more of claims 1 to 13, wherein a triarylphosphine containing from 1 to 6, in particular from 1 to 3, $-SO_3M$ groups, where the aryl radicals are identical or different and are each phenyl or naphthyl, or a mixture of such triarylphosphines, is used.

15. The process as claimed in one or more of claims 1 to 14, wherein a triphenylphosphine containing from 1 to 3 $-SO_3M$ groups is used.

16. The process as claimed in one or more of claims 1 to 13, wherein the phosphine used is one of the formula (A)

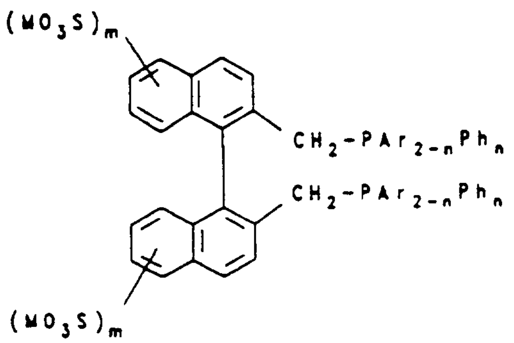

(A)

where Ar is $m-C_6H_4SO_3M$, M is hydrogen, ammonium, a monovalent metal or the equivalent of a polyvalent metal and Ph is a phenyl radical, m are identical or different and each have the value 1 or 2 and n are identical or different and are each 0, 1 or 2, or a mixture of such phosphines.

17. The process as claimed in one or more of claims 1 to 13, wherein the phosphine used is one of the formula (B)

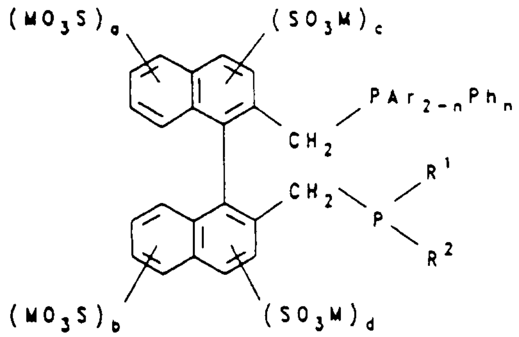

(B)

where M is hydrogen, ammonium, a monovalent metal or the equivalent of a polyvalent metal, a, b, c and d are each 0 or 1, with the proviso that a+b+c+d is an integer from 1 to 4, Ar is an m-($MSO_3$)-$C_6H_4$ radical and M is as defined above, n is 0, 1 or 2, Ph is a phenyl radical, $R^1$ and $R^2$ are identical or different and are each an alkyl radical having from 1 to 10 carbon atoms or a cycloaliphatic radical having from 5 to 10 carbon atoms in the ring, or a mixture of such phosphines.

**18.** The process as claimed in one or more of claims 1 to 13 and 17, wherein a phosphine of the formula (B), where $R^1$ and $R^2$ are each a cyclohexyl radical, is used.

**19.** A catalyst for carrying out the process as claimed in one or more of claims 1 to 13 and 17 to 18, wherein the catalyst contains Pd and the phosphine of the formula (B) in a molar ratio of from 1:1 to 1:10, in particular from 1:2 to 1:6.

**20.** A catalyst for carrying out the process as claimed in one or more of claims 1 to 13 and 17 to 18, wherein the catalyst contains Pd and the phosphine of the formula (B), where $R^1$ and $R^2$ are each a cyclohexyl radical, in a molar ratio of from 1:1 to 1:10, in particular from 1:2 to 1:6.

**Revendications**

**1.** Procédé pour la préparation d'amines aromatiques, caractérisé en ce que l'on fait réagir un composé nitro aromatique de formule générale $R^1R^2R^3ArNO_2$, dans laquelle $R^1$, $R^2$ et $R^3$ sont identiques ou différents et représentent un atome d'hydrogène, un reste alkyle ou alcoxy avec 1 à 6 atomes de carbone, un reste alkyle halogéné avec 1 à 4 atomes de carbone, un atome d'halogène, -OH, -CHO, -CO-$R^4$, -$CO_2$-$R^4$, -CONH$R^4$, -CON($R^4$)$_2$, -S$R^4$, $R^4$ représentant un reste avec 1 à 6 atomes de carbone, -CN ou -CH=CH-$R^5$, $R^5$ représentant un atome d'hydrogène, un reste alkyle avec 1 à 4 atomes de carbone ou un reste phényle, et Ar est un reste aromatique, dans un mélange de solvants comportant de l'eau et un solvant organique insoluble dans l'eau, sur un catalyseur contenant du palladium et une phosphine hydrosoluble, avec du monoxyde de carbone, sous une pression de 5 à 300 bars et à une température de 50 à 200 °C et on sépare la phase aqueuse et la phase organique.

**2.** Procédé selon la revendication 1, caractérisé en ce que l'on utilise un composé de formule $R^1R^2R^3ArNO_2$, dans laquelle $R^1$, $R^2$ et $R^3$ représentent un atome d'hydrogène, un reste alkyle ou alcoxy avec 1 à 4 atomes de carbone, un reste alkyle halogéné avec 1 à 2 atomes de carbone, un atome d'halogène, -OH, -CO-$R^4$, -CN ou -CH=CH-$R^5$ en tant que composé nitroaromatique.

**3.** Procédé selon la revendication 1 ou 2, caractérisé en ce que l'on utilise un composé de formule $R^1R^2R^3ArNO_2$, dans laquelle au moins l'un des restes $R^1$, $R^2$ et $R^3$ représente un atome d'halogène, -OH, -CO-$R^4$, -CN ou -CH=CH-$R^5$ en tant que composé nitroaromatique.

**4.** Procédé selon une ou plusieurs des revendications 1 à 3, caractérisé en ce que l'on utilise un composé de formule $R^1R^2R^3ArNO_2$, dans laquelle un ou deux des restes $R^1$, $R^2$ et $R^3$ représente un atome d'halogène, -OH, -CO-$R^4$, -CN ou -CH=CH-$R^5$ en tant que composé nitroaromatique.

**5.** Procédé selon une ou plusieurs des revendications 1 à 4, caractérisé en ce que l'on utilise un composé de formule $R^1R^2R^3ArNO_2$, dans laquelle un ou deux des restes $R^1$, $R^2$ et $R^3$ représente -Cl, -Br, -OH, -CO-$R^4$, $R^4$ représentant

un reste alkyle avec 1 à 4 atomes de carbone ou un reste phényle, -CN ou $-CH=CH-R^5$ $R^5$ représentant un atome d'hydrogène, un reste alkyle avec 1 à 4 atomes de carbone ou un reste phényle, en tant que composé nitroaromatique.

6. Procédé selon une ou plusieurs des revendications 1 à 5, caractérisé en ce que l'on utilise un composé de formule $R^1R^2R^3ArNO_2$, dans laquelle Ar représente un reste aromatique avec 6 à 20 atomes de carbone, en tant que composé nitroaromatique.

7. Procédé selon une ou plusieurs des revendications 1 à 6, caractérisé en ce que l'on utilise un composé de formule $R^1R^2R^3ArNO_2$, dans laquelle Ar représente un reste phényle, naphtyle, phénanthryle, biphényle ou binaphtyle en tant que composé nitroaromatique.

8. Procédé selon une ou plusieurs des revendications 1 à 7, caractérisé en ce que l'on utilise un composé de formule $R^1R^2R^3ArNO_2$, dans laquelle Ar représente un reste phényle, naphtyle ou biphényle en tant que composé nitroaromatique.

9. Procédé selon une ou plusieurs des revendications 1 à 8, caractérisé en ce que l'on utilise un composé de formule $R^1R^2R^3ArNO_2$, dans laquelle Ar représente un reste phényle en tant que composé nitro aromatique.

10. Procédé selon une ou plusieurs des revendications 1 à 9, caractérisé en ce que l'on utilise un hydrocarbure aromatique, un hydrocarbure aliphatique, un hydrocarbure chloré aromatique ou aliphatique, ou un mélange de ces hydrocarbures, en tant que solvants organiques insolubles dans l'eau.

11. Procédé selon une ou plusieurs des revendications 1 à 10, caractérisé en ce que l'on utilise du palladium sous forme d'un sel de palladium hydrosoluble, plus particulièrement $PdCl_2$, $Pd(NO_3)_2$, $(acétate)_2$ de Pd ou $PdSO_4$.

12. Procédé selon une ou plusieurs des revendications 1 à 11, caractérisé en ce que l'on utilise une phosphine contenant des groupes $-SO_3M$, où M représente un atome d'hydrogène, ammonium, un métal monovalent ou l'équivalent d'un métal multivalent, en tant que phosphines hydrosolubles.

13. Procédé selon une ou plusieurs des revendications 1 à 12, caractérisé en ce que l'on utilise une phosphine contenant des groupes $-SO_3M$, où M représente ammonium ou un métal alcalin, plus particulièrement le sodium ou le potassium, de préférence le sodium.

14. Procédé selon une ou plusieurs des revendications 1 à 13, caractérisé en ce-que l'on utilise une triarylphosphine contenant de 1 à 6, en particulier de 1 à 3 groupes $-SO_3M$, où le reste aryle est identique ou différent et représente phényle ou naphtyle, ou un mélange de telles triarylphosphines.

15. Procédé selon une ou plusieurs des revendications 1 à 14, caractérisé en ce que l'on utilise une triphénylphosphine contenant de 1 à 3 groupes $-SO_3M$.

16. Procédé selon une ou plusieurs des revendications 1 à 13, caractérisé en ce que l'on utilise une phosphine de formule générale (A)

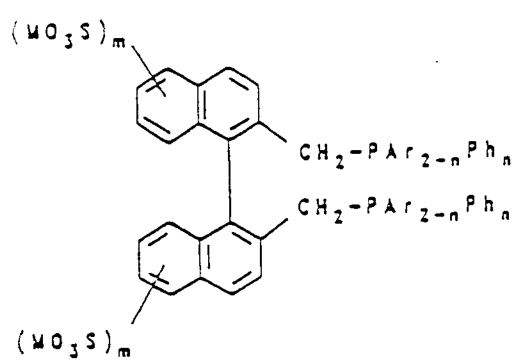

(A)

dans laquelle Ar représente m-$C_6H_4SO_3M$, M représente un atome d'hydrogène, ammonium, un métal monovalent ou l'équivalent d'un métal multivalent et Ph représente un reste phényle, m, identique ou différent, vaut 1 ou 2, et n, identique ou différent, vaut 0, 1 ou 2, ou un mélange de telles phosphines.

**17.** Procédé selon une ou plusieurs des revendications 1 à 13, caractérisé en ce que l'on utilise une phosphine de formule générale (B)

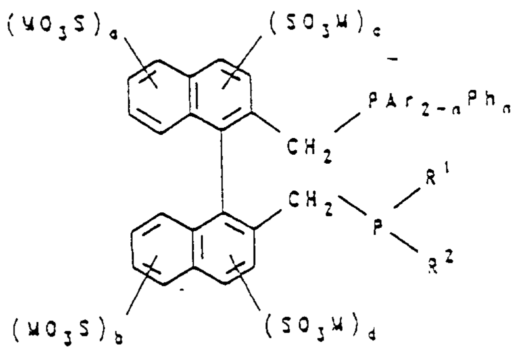

(B)

dans laquelle M représente un atome d'hydrogène, ammonium, un métal monovalent ou l'équivalent d'un métal multivalent, a, b, c et d valent chacun 0 ou 1, à la condition que a + b + c + d corresponde à un nombre entier de 1 à 4, Ar représente un reste m-($MSO_3$)-$C_6H_4$ et M a la signification donnée ci-dessus, n vaut 0, 1 ou 2, Ph représente un reste phényle, $R^1$ et $R^2$ sont identiques ou différents et représentent un reste alkyle avec 1 à 10 atomes de carbone ou un reste cycloaliphatique avec 5 à 10 atomes de carbone dans le cycle, ou un mélange de telles phosphines.

**18.** Procédé selon une ou plusieurs des revendications 1 à 13 at 17 caractérisé en ce que l'on utilise une phosphine de formule (B) dans laquelle $R^1$ et $R^2$ représentent chacun un reste cyclohexyle.

**19.** Catalyseur pour la mise en oeuvre du procédé selon une ou plusieurs des revendications 1 à 13 et 17 à 18, caractérisé en ce que le catalyseur contient du palladium et la phosphine de formule (B) dans un rapport molaire de 1:1 à 1:10, plus particulièrement de 1:2 à 1:6.

**20.** Catalyseur pour la mise en oeuvre du procédé selon une ou plusieurs des revendications 1 à 13 et 17 à 18, caractérisé en ce que le catalyseur contient du palladium et la phosphine de formule (B) dans laquelle $R^1$ et $R^2$ représentent chacun un reste cyclohexyle, dans un rapport molaire de 1:1 à 1:10, plus particulièrement de 1:2 à 1:6.